Europäisches Patentamt

European Patent Office

Office européen des brevets

(19)

(11) **EP 1 295 125 B1**

(12) **FASCICULE DE BREVET EUROPEEN**

(45) Date de publication et mention
de la délivrance du brevet:
**17.05.2006 Bulletin 2006/20**

(21) Numéro de dépôt: **01945401.6**

(22) Date de dépôt: **12.06.2001**

(51) Int Cl.:
***G01N 33/574*** *(2006.01)*

(86) Numéro de dépôt international:
**PCT/FR2001/001815**

(87) Numéro de publication internationale:
**WO 2001/096876 (20.12.2001 Gazette 2001/51)**

(54) **TEST IMMUNOLOGIQUE ET KIT DE DIAGNOSTIC D'UN ADENOCARCINOME DE LA PROSTATE**

IMMUNOLOGISCHES TEST UND SATZ ZUR DIAGNOSE VON PROSTATADENOKARZINOM

IMMUNOLOGICAL TEST AND DIAGNOSTIC KIT FOR PROSTATE ADENOCARCINOMA

(84) Etats contractants désignés:
**AT BE CH CY DE DK ES FI FR GB GR IE IT LI LU
MC NL PT SE TR**

(30) Priorité: **13.06.2000 FR 0007476**

(43) Date de publication de la demande:
**26.03.2003 Bulletin 2003/13**

(73) Titulaire: **Biomerieux S.A.
69280 Marcy l'Etoile (FR)**

(72) Inventeur: **CHARRIER, Jean-Philippe
F-69160 Tassin La Demi Lune (FR)**

(74) Mandataire: **Bonneau, Gérard
Murgitroyd & Company,
Immeuble Atlantis,
55, Allée Pierre Ziller
06560 Valbonne
Sophia Antipolis (FR)**

(56) Documents cités:
**WO-A-92/01936          WO-A-95/18381
WO-A-99/10745          WO-A-99/61914
DE-A- 4 322 342          FR-A- 2 780 791**

**Description**

La présente invention concerne une méthode de dépistage ou de diagnostic permettant de mettre en évidence la présence d'un cancer de la prostate dit adénocarcinome de la prostate, généralement abrégé sous la forme PCa, ou d'une hypertrophie bénigne de la prostate, dont l'abréviation est BPH, chez un patient, et ceci sans réaliser de biopsie. L'invention concerne plus généralement une méthode d'analyse en vue du diagnostic, du pronostic ou du suivi thérapeutique d'un cancer chez un patient humain.

Par diagnostic il faut comprendre que le marqueur permet de déterminer si le patient est atteint par une pathologie donnée (ou s'il n'est pas atteint). Par pronostic il faut comprendre que le marqueur permet de graduer la gravité de la pathologie. Le pronostic peut orienter les choix thérapeutiques. Enfin par suivi thérapeutique il faut comprendre que le marqueur permet de suivre l'efficacité du traitement. En cas d'échec du traitement (révélé par le marqueur), un traitement alternatif peut être proposé au patient.

Le PSA est produit par l'épithélium glandulaire de la prostate humaine, probablement sous une forme zymogène inactive (Lundwall *et al.* FEBS LeH 1987), et est sécrété dans le liquide séminal sous sa forme active (Lilja, J. Clin Invest 1985). L'activité biologique du PSA dans le liquide séminal est liée à sa fragmentation protéolytique limitée des protéines prédominantes sécrétées par les vésicules séminales (Lilja, J. Clin Invest 1985 ; Lilja *et al.* J. Clin Invest 1987 ; Mc Gee *et al.* Biol. reprod. 1988).

Le PSA est le principal marqueur du cancer de la prostate qui affectera, au cours de sa vie, un homme sur six en Occident. Cette protéase de la famille des kallikréines, principalement sécrétée par l'épithélium prostatique, est retrouvée à une concentration de 0,5 à 5 mg/ml dans le liquide séminal et à une concentration un million de fois moindre dans le sérum d'un patient. Ainsi, le PSA est trouvé normalement à une concentration inférieure à 2,5 ng/ml dans le sérum. Cependant, cette concentration augmente en principe notablement lors d'un cancer de la prostate et modérément lors d'altérations bénignes telles que l'hypertrophie de la prostate bénigne (BPH) ou la prostatite aiguë.

La séquence protéique du PSA a été déterminée. Il s'agit d'une glycoprotéine comportant 237 acides aminés ("Molecular cloning of human prostate specific antigen cDNA". Lundwall A., Lilja H., 1987. *FEBS Lett* 214: 317-322).

Cependant, la zone de recouvrement entre les différentes pathologies est responsable d'un manque important de sensibilité et de spécificité. C'est ainsi que 30 à 45 % des cancers confinés à la glande, qui constitue un stade précoce et potentiellement curable, ne sont pas dépistés avec le seuil usuel de 4 ng/ml alors que trois patients sur quatre sont suspectés à tort.

Par ailleurs, il a été montré récemment que dans le sérum, le PSA s'associait à des inhibiteurs de protéase, tels que l'$\alpha$-1-antichymostrypsine (ACT), et que l'usage du ratio PSA libre sur PSA total permettait d'améliorer la spécificité du diagnostic.

L'ACT est une glycoprotéine plasmatique de 68.000 Da dont la séquence est connue (Chandra T., Stackhouse R., Kidd V. J., Robson K. J., Woo S. L. ; Biochemistry 1983 Oct. 25 ; 22 (22): 5055-5061 ; Sequence homology between human $\alpha$-1-antichymotrypsin, $\alpha$-1-antitrypsin, and antithrombin III). Elle appartient à la famille des Serpines et inhibe de nombreuses protéases à Sérine, telle l'$\alpha$-1-antichymotrypsine ou le PSA. Sa concentration sanguine augmente en cas de dommage cellulaire suite à une blessure, une intervention chirurgicale ou un infection. Elle fait partie des protéines caractéristiques de la réponse inflammatoire. L'intérêt de dosage de l'ACT a été décrit dans le cas de nombreuses pathologies, telles que l'arthrite rhumatoïde, la maladie d'Alzheimer, la maladie d'Hodgkin, les cancers gastriques... Mais l'intérêt du dosage de l'ACT sérique n'a pas été démontré à ce jour pour le diagnostic du cancer de la prostate. L'état de la technique pousse même l'homme du métier à ne pas s'y intéresser, puisque les ratios à base de concentration de PSA sont les seuls préconisés.

L'art antérieur montre donc qu'il existe des techniques permettant de diagnostiquer le développement d'un cancer de la prostate chez les patients. Ainsi, la demande de brevet WO-A-97/12245 revendique une méthode permettant de diagnostiquer un adénocarcinome de la prostate sans biopsie. Cette méthode consiste à mesurer, dans le sérum ou dans le sang des patients, la quantité totale de PSA. Si cette valeur est comprise entre 2,5 et 20 ng/ml, on mesure également la concentration de PSA libre, On calcule ensuite le ratio PSA libre sur PSA total. Si ce ratio est inférieur à 7 %, le diagnostic s'oriente vers un adénocarcinome de la prostate.

Toutefois, l'utilisation d'un seuil à 7 %, pour le diagnostic d'un cancer de la prostate, est controversée par de nombreux auteurs, comme le montre la publication de Lein et al, « Relation of free PSA/total PSA in serum for differentiating between patients with prostatic cancer and benign prostate hyperplasia : which cutoff should be used ? ». Dans ce document publié dans la revue Cancer Investigation, 16(1), 45-49, 1998, il a été démontré qu'il est difficile, par le biais de ce ratio, de différencier systématiquement un cancer ou adénocarcinome de la prostate, d'une hypertrophie bénigne de la prostate.

En outre la publication de Catalona et al., « Prostate Cancer Detection in Men With Serum PSA Concentrations of 2,6 to 4,0 ng/ml and Benign Prostate Examination », publiée dans JAMA du 14 Mai 1997-Vol 277, No, 18, démontre qu'une concentration inférieure à 4 ng/ml doit être prise en considération pour dépister précocement un cancer de la prostate.

La demanderesse a décrit, dans une demande de brevet WO-A-00/02052, une méthode de diagnostic qui permet de combler le manque d'efficacité des tests dans la zone de concentration de PSA totale supérieur à 2 ng/ml. A cette fin, les formes moléculaires de PSA sérique de patients atteints de cancer ou de BPH ont été cartographiées par électrophorèse bi-dimensionnelle, associé à la détection par chimiluminescence, afin d'observer l'ensemble des formes de PSA libre, complexé et clivé.

Ainsi, les profils de sérum de cancéreux sont relativement homogènes, alors que ceux de BPH peuvent comporter une proportion relativement importante de formes clivées, et des spots légèrement plus basiques.

Il est donc démontré que l'augmentation du ratio PSA libre sur PSA total en cas de BPH peut être lié à l'existence de PSA clivé, enzymatique ment inactif et incapable de se lier à l'ACT, ou être en relation avec du PSA libre légèrement basique qui pourrait correspondre à la forme zymogène inactive.

Dans une autre demande de brevet FR00/04591, la demanderesse a mis au point des tests immunologiques pour mettre en oeuvre la méthode de diagnostic décrite dans WO-A-00/02052.

Pourtant, l'utilisation d'anticorps dirigés contre le PSA libre clivé ou non clivé est beaucoup plus difficile car il n'existe pas beaucoup d'anticorps commercialisés ou disponibles si l'on fait abstraction de ceux cités dans la demande de brevet FR00/04591. Ce n'est pas le cas d'autres paramètres, tels que PSA total (libre et complexé), PSA-ACT, PSA libre total (clivé et non clivé), qui sont des paramètres simples bien connus de l'état de la technique. Le ratio selon la présente invention est établi par le dosage de paramètres bien connus dans l'art antérieur. Il peut donc être réalisé simplement dans la plupart des laboratoires d'analyses médicales. WO 99/61914 décrit une métode pour différentier les patients ayant un adénocarcinome de la prostate et les patients souffrant d'une hyperplasie bénigne selon laquelle sont déterminées la concentration en PSA libre, PSA-ACT et PSA total.

Ainsi, le PSA complexé avec l'ACT est dosé par exemple à l'aide d'anticorps décrits dans la demande de brevet WO-A-98/22509.

Le PSA total est dosé par exemple à l'aide d'anticorps décrits par H. Nagasaki et al. (1999), Clin. Chem. 45 : 4486-4496, ou par l'intermédiaire d'anticorps commercialisés par la société Scripps Laboratories (San Diego,USA).

Le PSA libre est dosé par exemple à l'aide d'anticorps décrits dans la demande de brevet WO 92/01936, ou à l'aide d'anticorps monoclonaux commercialisés par la société Chagaï (Japon).

Enfin, l'ACT est dosé par exemple à l'aide d'anticorps décrits dans l'article de Sprecchia G., Petroboni V., Fratino P., Dander B., Introduction of antichymotrysin antibodies : their identification and interference with tryptic activity, Boll. Soc. Ital. Biol. Sper. 1970 Feb. 15 ; 46(3) : 111-4. Des anticorps permettant de doser l'ACT sont par ailleurs commercialisés par exemple par la société Dako (Trappes, France) ou la société Valbiotech (Paris, France). Une méthode de dosage a été par exemple décrite par Calvin J., Price C. P., Measurement of sérum alpha-1-antichymotrypsin by immunoturbidimetry, Ann. Clin. Biochem. 1986 ; 23 ; 206-209.

L'invention consiste donc à doser dans un liquide biologique le PSA libre, le PSA total (ou le PSA-ACT) et l'ACT, puis a réaliser le ratio suivant :

$$\frac{[ACT] \times [PSA\ total]^2}{[PSA\ libre]}$$ , qui est exprimé en $(\mu g/ml)^2$.

Le patient est diagnostiqué comme ayant un adénocarcinome de la prostate, si le ratio est supérieur à une première valeur, ou comme ayant une hypertrophie bénigne de la prostate, si le ratio est inférieur à une deuxième valeur.

De plus, par rapport à l'usage du ratio PSA libre / PSA total bien connu dans l'art antérieur, le ratio selon la présente invention permet d'augmenter de façon très significative la discrimination entre hypertrophie bénigne de la prostate et adénocarcinome de la prostate. Par conséquent, l'invention permet de réduire considérablement le nombre de biopsies nécessaires à la confirmation des pathologies malignes. Elle contribue donc a réduire les coûts de santé publique et les désagréments occasionnées aux patients lors d'une biopsie.

Enfin ce ratio cumule en une seule valeur l'effet de trois paramètres : le ratio PSA libre / PSA total, le taux de PSA total et le taux d'ACT. De ce fait, le clinicien dispose d'une seule valeur à analyser ce qui facilite son interprétation. Il n'a plus besoin d'analyser de façon concomitante le ratio PSA libre / PSA total et le taux de PSA total comme cela est pratiqué dans l'art antérieur. De surcroît, la valeur du ratio de la présente invention augmente en cas de cancer alors qu'elle est faible en cas de hypertrophie bénigne de la prostate. Cette valeur permet ainsi de graduer aisément l'avancé et la gravité du cancer.

A cet effet, la présente invention concerne une méthode d'analyse en vue du diagnostic, du pronostic ou du suivi thérapeutique d'un adénocarcinome de la prostate ou d'une hypertrophie bénigne de la prostate chez un patient humain mâle, sans pratiquer de biopsie prostatique, caractérisée en ce qu'elle utilise au moins un marqueur tissulaire et au moins un marqueur de l'inflammation pour le diagnostic.

Cette méthode consiste à utiliser le PSA libre, le PSA total ou le PSA-ACT comme marqueur tissulaire et au moins l'ACT comme marqueur de l'inflammation.

Selon un mode préférentiel de réalisation, la méthode consiste à utiliser la concentration d'au moins un et préférentiellement d'au moins deux des marqueur(s) tissulaire(s) suivants impliqués dans un ratio :

- [PSA libre] ;
- [PSA total] ; et
- [PSA-ACT].

Selon un autre mode préférentiel de réalisation la méthode consiste à utiliser la concentration de l'[ACT] impliquée dans un ratio comme marqueur de l'inflammation.

Dans tous les cas de figure et selon une première méthode, le ratio est le suivant :

$$\frac{[ACT] \times [PSA\ total]^2}{[PSA\ libre]}$$

Selon cette première méthode, lorsque le ratio est supérieur 30 et préférentiellement à 50 ($\mu$g/ml)2, on diagnostique un adénocarcinome de la prostate, et lorsque le ratio est inférieur à 16,5 et préférentiellement à 12 ($\mu$g/ml) 2, on diagnostique une hypertrophie bénigne de la prostate.

Dans tous les cas de figure et selon une deuxième méthode, le ratio est le suivant :

$$\frac{[ACT] \times [PSA - ACT] \times [PSA\ total]}{[PSA\ libre]}.$$

Selon cette deuxième méthode, lorsque le ratio est supérieur à 23 préférentiellement à 45 ($\mu$g/ml)2, on diagnostique un adénocarcinome de la prostate, et lorsque le ratio est inférieur à 13,5 et préférentiellement à 9 ($\mu$g/ml)2, on diagnostique une hypertrophie bénigne de la prostate.

Dans tous les cas de figure et selon une troisième méthode, le ratio est le suivant ou le ratio inverse :

$$\frac{[ACT] \times [PSA - ACT]^2}{[PSA\ libre]}$$

Selon cette troisième méthode, lorsque le ratio est supérieur à 17 préférentiellement à 40 ($\mu$g/ml)$^2$. on diagnostique un adénocarcinome de la prostate, et lorsque le ratio est inférieur à 12 et préférentiellement à 7 ($\mu$g/ml)$^2$. on diagnostique une hypertrophie bénigne de la prostate.

La présente invention concerne également un procédé utilisant une méthode, telle décrite ci-dessus, qui consiste à :

- déterminer la valeur des paramètres constituant le ratio qui doit être utilisé,
- déterminer la valeur du ratio,
- comparer le ratio trouvé par rapport aux valeurs de référence, et
- diagnostiquer soit un adénocarcinome de la prostate, soit une hypertrophie bénigne de la prostate, soit enfin une zone de flou impliquant de plus amples investigations, telles que biopsie, touché digital rectal, ultrasonographie trans-rectale.

L'invention concerne encore un test immunologique permettant de mettre en oeuvre un procédé, tel que décrit précédemment, qui utilise :

- un anticorps anti-PSA libre et au moins un autre anticorps choisi parmi les anticorps anti-PSA total et anti-PSA-ACT, et
- un anticorps anti-ACT.

Enfin l'invention concerne un kit de diagnostic permettant de diagnostiquer, de pronostiquer ou de suivre thérapeutiquement un adénocarcinome de la prostate chez un sujet soupçonné d'être atteint dudit adénocarcinome, ou permettant de différencier entre une pathologie bénigne de la prostate et un adénocarcinome de la prostate chez un sujet soupçonné d'être atteint par l'une de ces maladies, ledit kit comprenant :

- des moyens pour doser le PSA libre comme marqueur tissulaire et pour doser au moins un autre marqueur tissulaire choisi parmi le PSA total et le PSA-ACT, et
- des moyens pour doser l'ACT, en tant que marqueur de l'inflammation.

Préférentiellement, les moyens pour doser sont des anticorps.

Les figures ci-jointes sont données à titre d'exemple explicatif et n'ont aucun caractère limitatif. Elles permettront de mieux comprendre l'invention.

La figure 1 représente un diagramme mettant en évidence les cas d'hypertrophie bénigne de la prostate et d'adénocarcinome de la prostate, en comparant la concentration de PSA total (en abscisse) par rapport au pourcentage correspondant au ratio des concentrations de PSA libre sur PSA total (en ordonnée).

La figure 2 représente un diagramme mettant en évidence les cas d'hypertrophie bénigne de la prostate et d'adénocarcinome de la prostate, en comparant la concentration de PSA total (en abscisse) par rapport à la concentration d'ACT (en ordonnée).

La figure 3 représente un diagramme mettant en évidence les cas d'hypertrophie bénigne de la prostate et d'adénocarcinome de la prostate, en comparant la concentration d'ACT (en abscisse) par rapport au pourcentage correspondant au ratio des concentrations de PSA libre sur PSA total (en ordonnée).

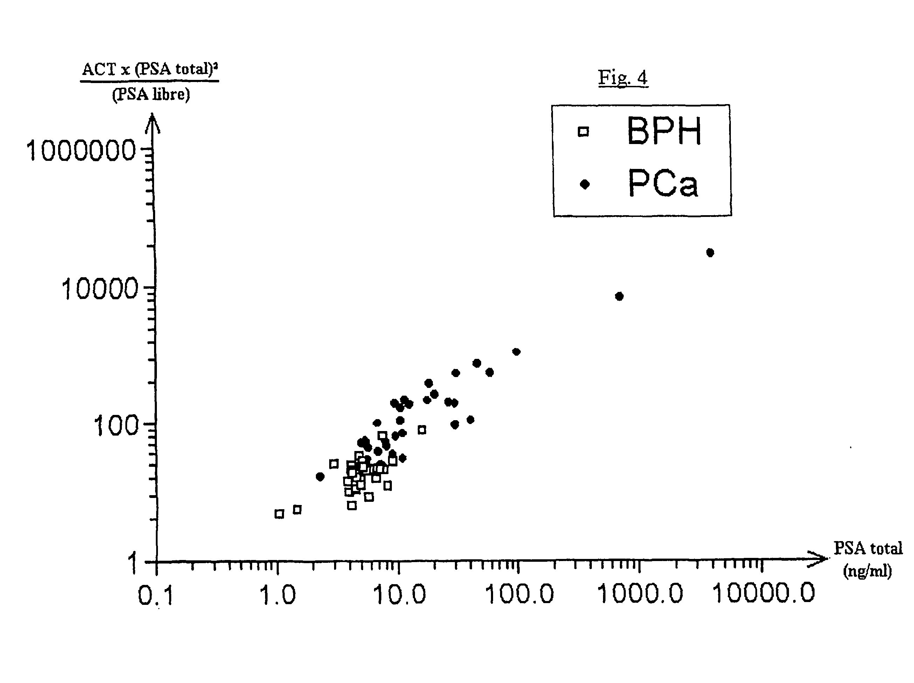

Enfin, la figure 4 représente un diagramme mettant en évidence les cas d'hypertrophie bénigne de la prostate et d'adénocarcinome de la prostate, en comparant la concentration de PSA total (en abscisse) par rapport à un ratio selon l'invention (en ordonnée).

L'intérêt du dosage du PSA total et du ratio PSA libre / total sont bien connu dans l'art antérieur, comme cela a été bien exposé précédemment. Par contre, l'intérêt du dosage de l'ACT n'a pas été démontré dans un but de diagnostic et de différenciation entre hypertrophie bénigne de la prostate et adénocarcinome de la prostate.

**A - Expériences réalisées :**

Le PSA libre a été dosé avec le kit FPSA Vidas (BioMérieux) selon les recommandations du fournisseur.

Le PSA total a été dosé avec le kit TPSA Vidas (BioMérieux) selon les recommandations du fournisseur.

L'ACT a été dosé par immunoturbidimètrie en adaptant une méthode décrite par J. Calvin et C. P. Price ("Mesure of serum $\alpha$1-antichymotrypsin by immunoturbidimetry" Ann, Clin, Biochem, 1986; 23: 206-209) selon le protocole suivant :

- Préparer le tampon A : Phosphate de Sodium 5 mM, NaCl 130 mM, Polyethylene glycol 6000 à 64 g/l, pH=7,2.
- Préparer la solution B: dilution de l'anticorps anti-ACT (Dako, référence A0022) à 1/16 en tampon A.
- Préparer une gamme étalon d'ACT par dilution d'ACT purifié (Scipac référence P159-5) en tampon A.
- Diluer les sérums à analyser à 1/10 en tampon A.
- Incuber la gamme et les sérums 15 minutes à température ambiante.
- Centrifuger la gamme et les sérums 10 minutes à 2000 g et 15°C.
- Mesurer la complexation de l'anticorps anti-ACT et de l'ACT en suivant l'augmentation de Densité Optique (D.O.) à 340 nm et 700 nm sur l'automate Hitachi 704. L'appareil est programmé pour dilué 18 $\mu$l d'échantillon dans 141 $\mu$l de tampon A, réaliser un blanc sérum puis ajouter 141 $\mu$l de solution B et lire la D.O. en point final.
- Etablir une courbe étalon en reportant les concentrations de la gamme étalon en fonction des D.O. obtenues.
- Calculer les taux d'ACT des échantillons en reportant les D.O. obtenues pour les échantillons sur la courbe étalon.

Le ratio suivant est calculé : $\dfrac{[ACT] \times [PSA\ total]^2}{[PSA\ libre]}$ (en $\mu$g/ml)$^2$ .

Le diagnostic du cancer de la prostate est porté si le ratio précédent est supérieur inférieur à 30 ($\mu$g/ml)$^2$.

Le diagnostic de hypertrophie bénigne de la prostate est porté si le ratio précédent est inférieur à 16,5 ($\mu$g/ml)$^2$.

Le présent ratio de la présente invention a été appliqué à l'étude de vingt -huit cas (28) de BPH et de trente-huit cas (38) de cancers de la prostate. Les résultats sont indiqués sur le tableau 1 ci-dessous.

Tableau 1 : Analyse de 28 cas de BPH et de 38 cas de PCa

| Patient | athologie | PSA total (ng/ml) | PSA libre (ng/ml) | ACT (μg/ml) | $\dfrac{[PSA\ libre]}{[PSA\ total]}$ (%) | $\dfrac{[ACT] \times [PSA\ total]^2}{[PSA\ libre]}$ |
|---|---|---|---|---|---|---|
| 1 | BPH | 1,04 | 0,20 | 861,40 | 19,23 | 4,66 |
| 2 | BPH | 1,47 | 0,35 | 875,20 | 23,81 | 5,40 |
| 3 | BPH | 3,01 | 0,32 | 879,00 | 10,63 | 24,89 |
| 4 | BPH | 8,27 | 3,50 | 616,30 | 42,32 | 12,04 |
| 5 | BPH | 7,50 | 0,83 | 926,30 | 11,07 | 62,78 |
| 6 | BPH | 3,90 | 0,87 | 796,80 | 22,31 | 13,93 |
| 7 | BPH | 16 | 2,24 | 661,50 | 14,00 | 75,60 |
| 8 | BPH | 9,13 | 2,84 | 946,00 | 31,11 | 27,77 |
| 9 | BPH | 6,22 | 1,15 | 634,90 | 18,49 | 21,36 |
| 10 | BPH | 4,01 | 0,52 | 317,50 | 12,97 | 9,82 |
| 11 | BPH | 5,8 | 1,86 | 465,00 | 32,07 | 8,41 |
| 12 | BPH | 5,03 | 0,46 | 279,10 | 9,15 | 15,35 |
| 13 | BPH | 4,55 | 1,01 | 518,50 | 22,20 | 10,63 |
| 14 | BPH | 4,85 | 0,41 | 578,70 | 8,45 | 33,20 |
| 15 | BPH | 4,17 | 0,71 | 969,10 | 17,03 | 23,73 |
| 16 | BPH | 4,52 | 1,05 | 568,30 | 23,23 | 11,06 |
| 17 | BPH | 6,81 | 1,02 | 472,70 | 14,98 | 21,49 |
| 18 | BPH | 4,16 | 0,73 | 877,30 | 17,55 | 20,80 |
| 19 | BPH | 5,55 | 1,00 | 669,40 | 18,02 | 20,62 |
| 20 | BPH | 4,6 | 0,79 | 599,80 | 17,17 | 16,07 |
| 21 | BPH | 4,23 | 0,44 | 458,40 | 10,40 | 18,64 |
| 22 | BPH | 6,71 | 1,56 | 536,60 | 23,25 | 15,49 |
| 23 | BPH | 7,67 | 2,57 | 911,70 | 33,51 | 20,87 |
| 24 | BPH | 5,12 | 0,54 | 559,60 | 10,55 | 27,17 |
| 25 | BPH | 4,18 | 2,28 | 804,00 | 54,55 | 6,16 |
| 26 | BPH | 5,01 | 1,17 | 586,90 | 23,35 | 12,59 |
| 27 | BPH | 7,21 | 1,43 | 585,10 | 19,83 | 21,27 |
| 28 | BPH | 5,24 | 0,72 | 595,70 | 13,74 | 22,72 |
| 29 | PCa | 2,31 | 0,31 | 976,10 | 13,42 | 16,80 |
| 30 | PCa | 12,47 | 0,81 | 983,80 | 6,50 | 188,87 |
| 31 | PCa | 46,46 | 3,43 | 1165,30 | 7,38 | 733,33 |
| 32 | PCa | 98,82 | 7,68 | 852,70 | 7,77 | 1084,24 |
| 33 | PCa | 58,82 | 6,84 | 1096,00 | 11,63 | 554,38 |
| 34 | PCa | 17,49 | 1,09 | 757,00 | 6,23 | 212,45 |
| 35 | PCa | 31,01 | 2,58 | 1453,70 | 8,32 | 541,82 |
| 36 | PCa | 29,83 | 1,73 | 377,30 | 5,80 | 194,06 |

Suite de tableau

| Patient | athologie | PSA total (ng/ml) | PSA libre (ng/ml) | ACT ($\mu$g/ml) | [PSA libre] / [PSA total] (%) | $\dfrac{[ACT] \times [PSA\ total]^2}{[PSA\ libre]}$ |
|---|---|---|---|---|---|---|
| 37 | PCa | 26,51 | 3,16 | 902,20 | 11,92 | 200,65 |
| 38 | PCa | 10,57 | 0,70 | 1059,80 | 6,62 | 169,15 |
| 39 | PCa | 10,55 | 2,09 | 2000,00 | 19,81 | 106,51 |
| 40 | PCa | 11,43 | 0,89 | 1496,50 | 7,79 | 219,67 |
| 41 | PCa | 40,39 | 10,00 | 668,10 | 24,76 | 108,99 |
| 42 | PCa | 7,9 | 1,79 | 1549,00 | 22,66 | 54,01 |
| 43 | PCa | 6,77 | 0,61 | 1311,70 | 9,01 | 98,56 |
| 44 | PCa | 5,56 | 0,86 | 835,20 | 15,47 | 30,02 |
| 45 | PCa | 10,84 | 2,56 | 662,70 | 23,62 | 30,42 |
| 46 | PCa | 713,00 | 104,00 | 1383,50 | 14,59 | 6762,77 |
| 47 | PCa | 11,00 | 1,21 | 703,00 | 11,00 | 70,30 |
| 48 | PCa | 10,5 | 0,84 | 801,30 | 8,00 | 105,17 |
| 49 | PCa | 9 | 2,61 | 1138,80 | 29,00 | 35,34 |
| 50 | PCa | 5,4 | 0,38 | 722,30 | 7,00 | 55,72 |
| 51 | PCa | 4000 | 567,00 | 1026,50 | 14,18 | 28966,49 |
| 52 | PCa | 9,5 | 0,34 | 734,40 | 3,58 | 194,94 |
| 53 | PCa | 9,58 | 1,23 | 853,30 | 12,84 | 63,67 |
| 54 | PCa | 7,07 | 1,24 | 606,20 | 17,54 | 24,44 |
| 55 | PCa | 4,09 | 0,70 | 907,90 | 17,11 | 21,70 |
| 56 | PCa | 6,81 | 0,92 | 762,50 | 13,51 | 38,44 |
| 57 | PCa | 18,41 | 1,64 | 1836,80 | 8,91 | 379,60 |
| 58 | PCa | 8,09 | 1,00 | 707,00 | 12,36 | 46,27 |
| 59 | PCa | 20,29 | 3,15 | 2000,00 | 15,52 | 261,39 |
| 60 | PCa | 29,98 | 7,15 | 727,00 | 23,85 | 91,39 |
| 61 | PCa | 7,39 | 1,18 | 508,30 | 15,97 | 23,52 |
| 62 | PCa | 5,42 | 0,84 | 790,90 | 15,50 | 27,66 |
| 63 | PCa | 5,66 | 0,70 | 967,70 | 12,37 | 44,29 |
| 64 | PCa | 5,42 | 0,30 | 546,90 | 5,54 | 53,55 |
| 65 | PCa | 4,98 | 0,29 | 596,30 | 5,82 | 50,99 |
| 66 | PCa | 4,63 | 0,71 | 776,00 | 15,33 | 23,43 |

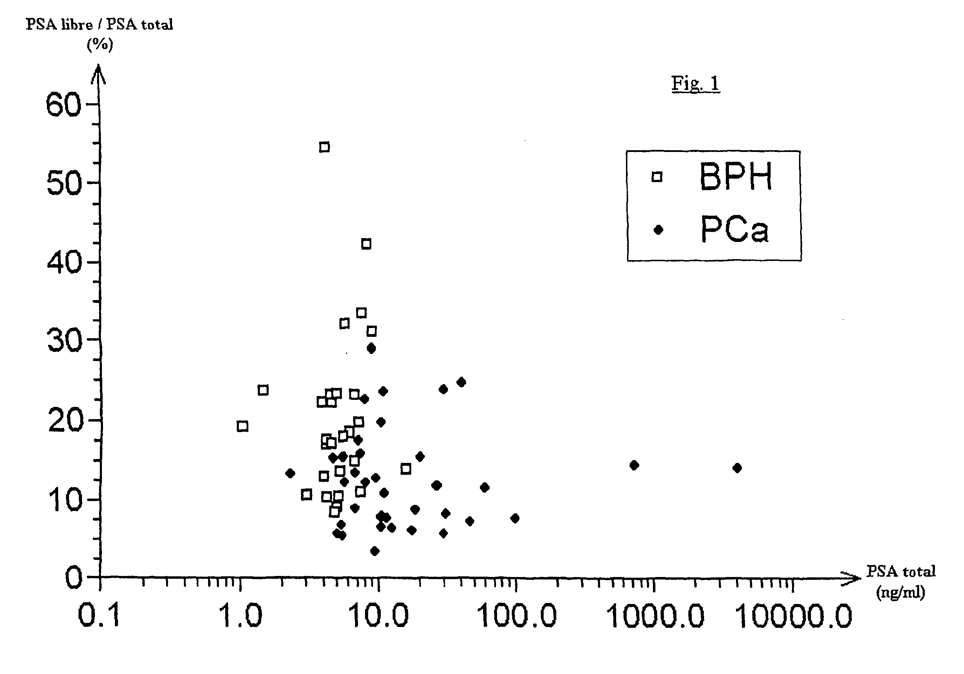

Les résultats obtenus sur le tableau 1 sont représentés sur les figures 1 à 4.

La figure 1 illustre le pouvoir de discrimination du taux de PSA total et du ratio PSA libre / PSA total, tel qu'il est utilisé dans l'art antérieur. La représentation montre que ces deux dosages ne sont pas corrélés et conduisent à des informations complémentaires et indépendantes.

Ainsi, si l'on reprend la valeur de 7 % qui a été mise en exergue lors de l'analyse de l'état de la technique, il faut constater que si tous les cas, dont le ratio est inférieur à 7 %, sont des cas de cancer, il n'en va pas de même au dessus puisqu'entre 7 et 30 % les adénocarcinomes de la prostate et les hypertrophies bénignes de la prostate sont parfaitement mélangés. Il n'y a qu'au dessus de 30 % que tous les cas sont des hypertrophies bénignes de la prostate.

Il y a donc une zone de flou qui est incompatible avec les problèmes de santé publique qui peuvent être générées. Il y a même une majorité de faux négatifs puisque vingt-quatre (24) des trente-huit (38) cas de cancer sont dans cette zone.

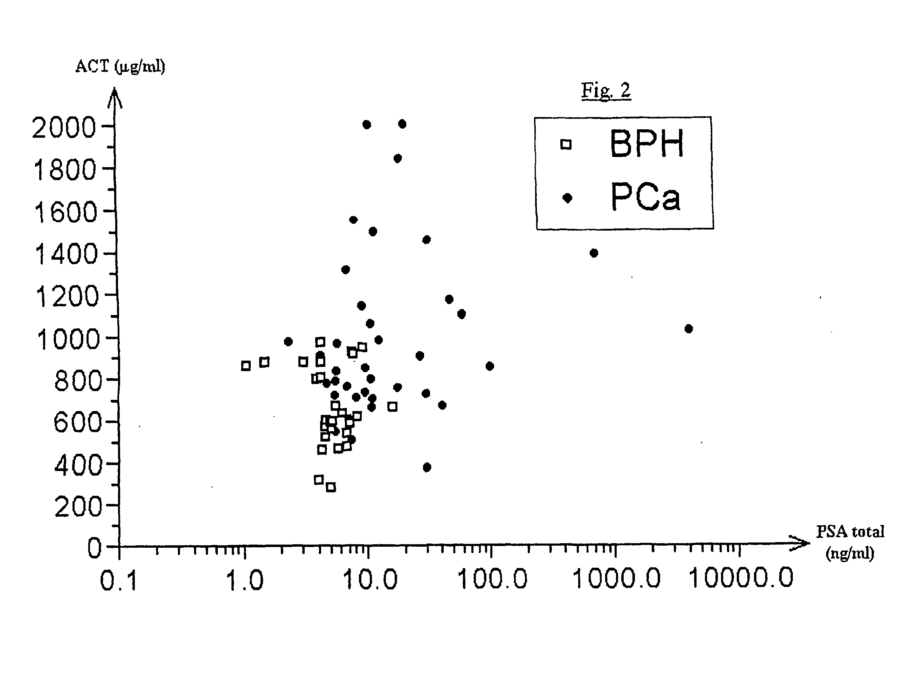

La figure 2 illustre le pouvoir de discrimination de l'ACT associé au PSA total. La représentation montre que ces deux dosages ne sont pas corrélés et conduisent à des informations complémentaires et indépendantes.

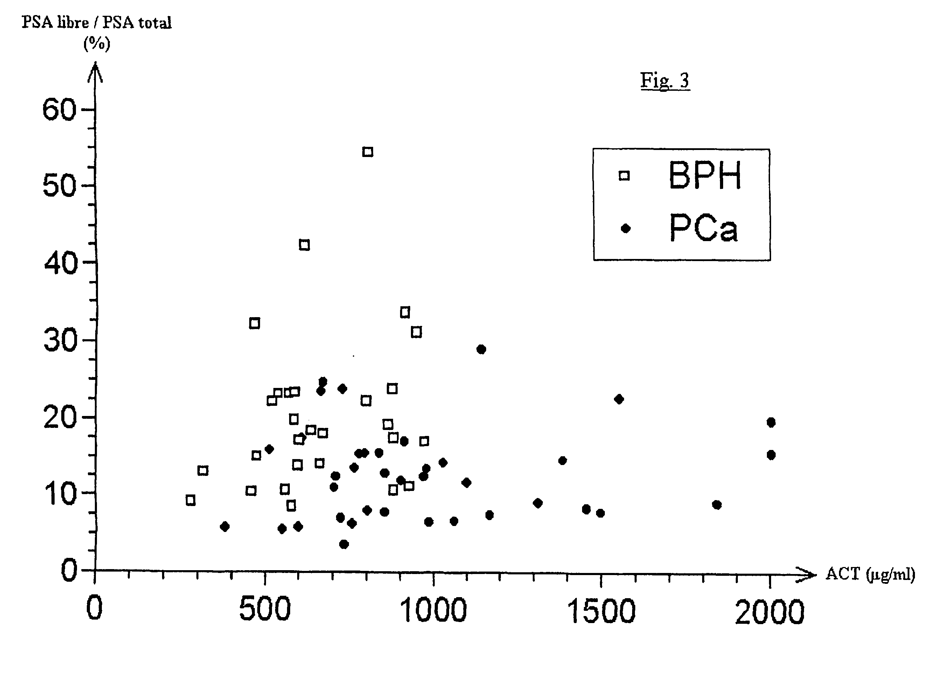

La figure 3 illustre le pouvoir de discrimination de l'ACT associé au ratio PSA libre / PSA total. La représentation montre que ces deux dosages ne sont pas corrélés et conduisent à des informations complémentaires et indépendantes.

Il résulte donc des figures 1 à 3 que les dosages de l'ACT, du PSA total et du PSA libre / PSA total conduisent à des informations complémentaires mais indépendantes. Il est donc impossible de tirer des conclusions lorsque l'on regarde ces diagrammes (figures 2 et 3) ou bien leur interprétation est difficile et délicate (figure 1).

L'association de ces trois paramètres en une seule valeur selon le ratio décrit dans la présente invention permet de manière surprenante d'associer la pertinence de chacun de ces paramètres pris séparément.

La figure 4 illustre le pouvoir de discrimination de l'invention caractérisé par usage du ratio en $(\mu g/ml)^2$:

$$\frac{[ACT] \times [PSA\ total]^2}{[PSA\ libre]}.$$

Elle montre à titre d'exemple la corrélation entre ledit ratio et le taux de PSA total,

Les quatre tableaux 2 à 5 qui suivent illustrent l'intérêt de l'invention par rapport à l'usage du ratio PSA libre / total bien connu dans l'art antérieur.

| $\dfrac{[PSA\ libre]}{[PSA\ total]}$ | ≤ 7 % | 7 à 25 % | ≥ 25 % |
|---|---|---|---|
| PCa | 8/38 = 21,1% - | 29/38 = 76,3 % | 1/38 = 2,6% |
| BPH | 0/28=0% | 23/28 = 82,1% | 5/28=17,9% |

Tableau 2: Pouvoir discriminant du ratio $\dfrac{[PSA\ libre]}{[PSA\ total]}$ en utilisant les seuils définis dans la demande de brevet WO-A-97/12245

| $\dfrac{[PSA\ libre]}{[PSA\ total]}$ | ≤ 7% | 7 à 25 % | ≥25 % |
|---|---|---|---|
| PSA total | ≥ 10 ng/ml | > 4 mais < 10 ng/ml | ≤ 4 ng/ml |
| PCa | 24/38=63,2% | 12/38=31,6% | 2/38=5,3% |
| BPH | 1/28=3,6% | 18/28=64,3% | 9/28=32,1% |

Tableau 3 : Pouvoir discriminant du ratio $\dfrac{[PSA\ libre]}{[PSA\ total]}$ avec les seuils précédents associés aux seuils de PSA total

| $\dfrac{[PSA\ libre]}{[PSA\ total]}$ | ≤ 10,4 % | 10,4 à 29 % | ≥29% |
|---|---|---|---|
| PCa | 15/38=39,5% | 22/38=57,9% | 0/38=0% |
| BPH | 2/28=7,1% | 21/28=75% | 5/28=17,9% |

Tableau 4 : Pouvoir discriminant du ratio $\dfrac{[\text{PSA libre}]}{[\text{PSA total}]}$ en utilisant des seuils optimisés de façon comparables

à ceux du $\dfrac{[\text{ACT}] \times [\text{PSA total}]^2}{[\text{PSA libre}]}$

| $\dfrac{[\text{ACT}] \times [\text{PSA total}]^2}{[\text{PSA libre}]}$ | $\geq 30\ (\mu g/ml)^2$ | $16,5$ à $30\ (\mu g/ml)^2$ | $\leq 16,5\ (\mu g/ml)^2$ |
|---|---|---|---|
| PCa | 32/38=84,2% | 6/38=15,8% | 0/38=0% |
| BPH | 3 / 28 = 10,7 % | 12 / 28 = 42,9 % | 13 / 28 = 46,4 % |

Tableau 5 : Pouvoir discriminant du ratio $\dfrac{[\text{ACT}] \times [\text{PSA total}]^2}{[\text{PSA libre}]}$ en utilisant des seuils optimisés

On remarque aisément que par rapport aux meilleurs critères utilisés dans l'art antérieur (tableau 4), le ratio proposé par la présente invention (tableau 5) permet d'augmenter très significativement le nombre de cas de cancer et de BPH correctement diagnostiqués (respectivement 32 contre 24 et 13 contre 9) et par la même occasion de réduire considérablement la zone d'incertitude (18 contre 30).

Le nombre de faux diagnostics est identique dans les deux cas, puisqu'il y a, pour notre invention, trois (3) cas qui sont classés comme hypertrophies bénignes de la prostate suite à une biopsie, alors que le ratio selon notre présente invention établissait des adénocarcinomes de la prostate. Dans le cas de ratios, selon l'état de la technique, deux (2) cas de cancers par biopsie sont en fait des hypertrophies bénignes de la prostate et une (1) hypertrophie bénigne de la prostate est en fait une adénocarcinome de la prostate pour l'art antérieur.

Même s'il y a le même nombre de faux positifs, notre ratio est plus favorable, car nous n'avons pas de cas de patient ayant un adénocarcinome de la prostate et qui n'est pas dépisté, alors qu'il y a deux cas selon l'état de la technique.

Il faut noter que contrairement à fart antérieur (tableaux 2 et 3), la présente invention ne diagnostique aucun cas de cancer de façon erronée. Cette situation est beaucoup plus fâcheuse que le diagnostic erroné de hypertrophie bénigne de la prostate qui est possible, quoique rare, dans la présente invention (2 sur 66), En effet, un diagnostic de cancer de la prostate est classiquement confirmé par biopsie ce qui minimise le risque de diagnostiquer à tort un cancer.

En revanche, aucune biopsie n'est pratiquée dans le cas d'un diagnostic de hypertrophie bénigne de la prostate. En conséquence, une personne atteinte d'un cancer et faussement diagnostiquée comme souffrant d'une hypertrophie bénigne de la prostate ne subira pas de thérapie adéquate. Son cancer risque alors d'être diagnostiqué ultérieurement, à un stade plus avancé, ce qui réduit ses chances de guérison et son espérance de vie.

## B - Expériences réalisables et éventuellement réalisées :

Toute autre méthode de dosage des trois paramètres (PSA libre, PSA total et ACT) est utilisable. A titre d'exemple, nous avons également mis en oeuvre un dosage de l'ACT par immunoélectrophorèse et par ELISA.

Ce protocole de dosage de l'ACT par immunoélectrophorèse a été réalisé selon la méthode de C. B. Laurell (Anal. Biochem, 1966, 15, 45-52) et adaptant une technique décrite par J. Calvin et C. P. Price ("Mesure of serum $\alpha$1-antichymotrypsin by immunoturbidimetry" Ann. Clin. Biochem, 1986 ; 23: 206-209). Ce protocole consiste en les étapes qui suivent :

- Préparer le tampon C : Veronal acide 1,842 g/l, Veronal sodée 10,3 g/l, acétate de sodium 5,8 g/l, pH=8,6.
- Préparer la solution D : NaCl 9 g/l, bleu de Bromophénol 0,01 %.
- Préparer la solution E : Dissoudre 10 g/l d'Agarose dans le tampon C (dilué au 1/4) en chauffant la solution au bain marie puis la laisser refroidir à 55°C. Ajouter 147 $\mu$l d'anticorps anti-ACT (Dako, référence A0022) pour 22 ml de solution d'agarose.
- Préparer la solution F : Ethanol 25%, acide Acétique 10%, eau déminéralisée 65%.
- Préparer la solution G : Bleu de Coomassie R-250 dilué à 1,15 g/l en solution F.
- Couler 22 ml de solution E à 55°C sur une plaque de 10x13 cm de gel bond Agarose (Pharmacia, référence 80-1129-32). Laisser refroidir et gélifier.
- Réaliser des puits de 2 mm de diamètre sur le bord du gel de 13 cm.

- Installer le gel sur une cuve d'immunoélectrophorèse (Gelman Science, Deluxe Electrophoresis Chamber) avec des mèches appliquées sur les bords du gel et trempant dans le tampon C.
- Préparer une gamme étalon d'ACT par dilution d'ACT purifié (Scipac référence P159-5) en tampon D.
- Diluer les sérums au 1/10 en tampon D.
- Déposer sur le gel 2 $\mu$l d'échantillon ou de point de gamme par puits.
- Laisser migrer environ 1 heure 10 minutes à 200 V et 400 mA.
- Sécher le gel par buvardage puis à l'étuve à 37°C.
- Colorer 1 minute avec la solution G.
- Décolorer avec la solution F jusqu'à obtenir un bruit de fond acceptable.
- Mesurer la hauteur des arcs de précipitation (rockets) visibles sur le gel.
- Etablir une courbe étalon en reportant les concentrations de la gamme étalon en fonction des hauteurs obtenues.
- Calculer les taux d'ACT des échantillons en reportant les hauteurs obtenues pour les échantillons sur la courbe étalon.

Il est également possible d'effectuer un protocole de dosage de l'ACT par ELISA sur microplaque. Dans ce cas le protocole consiste dans les étapes suivantes :

- Préparer le tampon H : Tris(hydroxymethyl)-aminomethane 0,2 M, Acide Maléique 0,2 M, pH=6,2.
- Préparer la solution I : tampon H plus 5% de caséine hydrolysée.
- Préparer le tampon J : tampon H plus 0,5% de tween 20.
- Préparer la solution K : Tris(hydroxymethyl)-aminomethane 0,1 M, NaCl 0,1 M, $MgCl_2$ 1 mM, $ZnCl_2$ 0,1 mM, azide de sodium 0,9 g/l, pH=6,5.
- Diluer l'anticorps anti-ACT (Dako, référence A0022) à 1,86 $\mu$g/ml en tampon H.
- Répartir 100 $\mu$l de solution d'anticorps anti-ACT par puits de microplaque. Incuber 1 heure à 37°C. Aspirer la solution.
- Répartir 200 $\mu$l de solution I. Incuber 1 heure à 37°C. Aspirer la solution.
- Préparer une gamme étalon d'ACT par dilution d'ACT purifié (Scipac référence P159-5) en tampon H.
- Diluer les sérums à 1/500 en tampon H.
- Incuber 100 $\mu$l d'échantillon ou de point de gamme par puits 1 heure à 37°C.
- Laver par 3 x 300 $\mu$l de tampon J.
- Diluer le conjugué anti-ACT couplé à la phosphatase alcaline (bioMérieux) dans la solution K au 1/500.
- Incuber 100 $\mu$l de conjugué par puits 1 heure à37°C.
- Laver par 3 x 300 $\mu$l de tampon J.
- Révéler avec 100 $\mu$l de substrat para-Nitro-Phenyl-Phosphate (pNPP).
- Stopper la révélation par 100 $\mu$l de soude 1M.
- Lire sur photospectromètre à 405 nm avec soustraction du bruit de fond à 630 nm.
- Etablir une courbe étalon en reportant les concentrations de la gamme étalon en fonction des D.O. obtenues.
- Calculer les taux d'ACT des échantillons en reportant les D.O. obtenues pour les échantillons sur la courbe étalon,

## C - Modifications possibles de la méthode :

Il est également possible de mesurer le PSA-ACT en lieu et place du PSA total et d'utiliser le ratio suivant :

$$\frac{[ACT] \times [PSA - ACT]^2}{[PSA \; libre]} \; en \; (\mu g/ml)^2.$$

Le patient est diagnostiqué comme ayant un adénocarcinome de la prostate, si le ratio est supérieur à une première valeur, ou comme ayant une hypertrophie bénigne de la prostate, si le ratio est inférieur à une deuxième valeur.

Il est également possible d'utiliser la concentration en PSA-ACT et en PSA total et d'utiliser le ratio suivant :

$$\frac{[ACT] \times [PSA - ACT] \times [PSA \; total]}{[PSA \; libre]} \; en \; (\mu g/ml)^2.$$

L'inversion des ratios conduit bien évidemment aux mêmes diagnostics que les ratios représentés dans l'invention à condition d'adapter les seuils aux nouveaux calculs.

De façon intéressante, l'invention permet de diagnostiquer un cancer de la prostate sans utiliser de marqueur tumoraux stricto sensu. En effet, le PSA et l'ACT sont aussi bien exprimés chez un individu sain que chez un individu atteint d'un cancer de la prostate. En fait, le diagnostic repose ici sur l'association d'un marqueur tissulaire (le PSA) et d'un marqueur de l'inflammation (l'ACT). Or le seul marqueur à visée diagnostique utilisé actuellement en cancérologie est le PSA.

De nombreux marqueurs tumoraux ont été décrits, mais aucun ne permet un dépistage précoce de la maladie, leur utilisation est généralement limité à un usage pronostique ou de suivi thérapeutique. L'invention démontre donc l'intérêt de l'association d'un marqueur tissulaire à un marqueur de l'inflammation pour le diagnostic d'un type de cancer. Une approche comparable est possible pour le dépistage d'autre type de cancer, ce qui serait extrêmement utile.

## Revendications

1. Méthode d'analyse en vue du diagnostic, du pronostic ou du suivi thérapeutique d'un adénocarcinome de la prostate ou d'une hypertrophie bénigne de la prostate chez un patient humain mâle, sans pratiquer de biopsie prostatique, **caractérisée en ce qu'**elle utilise au moins un marqueur tissulaire choisi parmi le PSA libre, le PSA total et le PSA-ACT et au moins l'ACT comme marqueur de l'inflammation, pour le diagnostic.

2. Méthode, selon la revendication 1, **caractérisée en ce qu'**elle consiste à utiliser la concentration d'au moins un et de préférence d'au moins deux marqueur(s) tissulaire(s) impliqué(s) dans un ratio, lesdits marqueurs tissulaires étant choisis parmi :

   • [PSA libre],
   • [PSA total], et
   • [PSA-ACT].

3. Méthode, selon la revendications 1, **caractérisée en ce qu'**elle consiste à utiliser la concentration de l'[ACT] impliquée dans un ratio comme marqueur de l'inflammation.

4. Méthode, selon l'une quelconque des revendications 1 à 3, **caractérisée en ce que** le ratio est le suivant :

$$\frac{[ACT] \times [PSA\ total]^2}{[PSA\ libre]}.$$

5. Méthode, selon la revendication 4, **caractérisée en ce que** lorsque le ratio est supérieur 30 et préférentiellement à 50 $(\mu g/ml)^2$, on diagnostique un adénocarcinome de la prostate, et lorsque le ratio est inférieur à 16,5 et préférentiellement à 12 $(\mu g/ml)^2$, on diagnostique une hypertrophie bénigne de la prostate.

6. Méthode, selon l'une quelconque des revendications 1 à 3, **caractérisée en ce que** le ratio est le suivant :

$$\frac{[ACT] \times [PSA - ACT] \times [PSA\ total]}{[PSA\ libre]}.$$

7. Méthode, selon la revendication 6, **caractérisée en ce que** lorsque le ratio est supérieur 23 préférentiellement à 45 $(\mu g/ml)^2$, on diagnostique un adénocarcinome de la prostate, et lorsque le ratio est inférieur à 13,5 et préférentiellement à 9 $(\mu g/ml)^2$, on diagnostique une hypertrophie bénigne de la prostate.

8. Méthode, selon l'une quelconque des revendications 1 à 3, **caractérisée en ce que** le ratio est le suivant ou le ratio inverse :

$$\frac{[ACT] \times [PSA - ACT]^2}{[PSA\ libre]}.$$

**9.** Méthode, selon la revendication 8, ***caractérisée en ce*** **que** lorsque le ratio est supérieur 17 préférentiellement à 40 $(\mu g/ml)^2$, on diagnostique un adénocarcinome de la prostate, et lorsque le ratio est inférieur à 12 et préférentiel-lement à 7 $(\mu g/ml)^2$, on diagnostique une hypertrophie bénigne de la prostate.

**10.** Procédé utilisant une méthode, selon l'une quelconque des revendications 1 à 9, **caractérisé en ce qu'**il consiste à :

- déterminer la valeur des paramètres constituant le ratio qui doit être utilisé,
- déterminer la valeur du ratio,
- comparer le ratio trouvé par rapport aux valeurs de référence, et
- diagnostiquer soit un adénocarcinome de la prostate, soit une hypertrophie bénigne de la prostate, soit enfin une zone de flou impliquant de plus amples investigations, telles que biopsie, touché digital rectal, ultrasonographie trans-rectal.

**11.** Test immunologique permettant de mettre en oeuvre un procédé, selon la revendication 10, qui utilise :

• un anticorps anti-PSA libre et au moins un autre anticorps choisi parmi les anticorps anti-PSA total et anti-PSA-ACT, et
• un anticorps anti-ACT.

**12.** Kit de diagnostic permettant de diagnostiquer, pronostiquer ou suivre thérapeutiquement un adénocarcinome de la prostate chez un sujet soupçonné d'être atteint dudit adénocarcinome, ou permettant de différencier entre une pathologie bénigne de la prostate et un adénocarcinome de la prostate chez un sujet soupçonné d'être atteint par l'une de ces maladies, ledit kit comprenant :

• des moyens pour doser le PSA libre comme marqueur tissulaire et pour doser au moins un autre marqueur tissulaire choisi parmi le PSA total et le PSA-ACT, et
• des moyens pour doser l'ACT, en tant que marqueur de l'inflammation.

**13.** Kit selon la revendication 12, dans lequel lesdits moyens sont des anticorps.

**Claims**

**1.** An method for analysis with a view to diagnosing, prognosing or therapeutically monitoring an adenocarcinoma of the prostate or a benign hypertrophy of the prostate in a human male patient, without carrying out a prostatic biopsy, **characterised in that** it uses at least one tissue marker, selected from free PSA, total PSA and PSA-ACT, and at least ACT as an inflammation marker, for diagnosis.

**2.** The method according to Claim 1, **characterised in that** it consists in using the concentration of at least one, and preferably of at least two, tissue marker(s) which are involved in a ratio, said tissue markers being selected from:

• [free PSA],
• [total PSA], and
• [PSA-ACT].

**3.** The method according to Claim 1, **characterised in that** it consists in using the concentration of the [ACT], which is involved in a ratio, as an inflammation marker.

**4.** The method according to any one of Claims 1 to 3, **characterised in that** the ratio is the following:

$$\frac{[ACT] \times [total\ PSA]^2}{[free\ PSA]}\ .$$

**5.** The method according to Claim 4, **characterised in that**, when the ratio is higher than 30, and preferably higher than $50(\mu g/ml)^2$, an adenocarcinoma of the prostate is diagnosed, and when the ratio is lower than 16.5, and preferably lower than 12$(\mu g/ml)^2$, a benign hypertrophy of the prostate is diagnosed.

6. The method according to any one of Claims 1 to 3, **characterised in that** the ratio is the following:

$$\frac{[ACT] \times [PSA - ACT] \times [total\ PSA]}{[free\ PSA]}.$$

7. The method according to Claim 6, **characterised in that**, when the ratio is higher than 23, and preferably higher than 45$(\mu g/ml)^2$, an adenocarcinoma of the prostate is diagnosed, and when the ratio is lower than 13.5, and preferably lower than 9$(\mu g/ml)^2$, a benign hypertrophy of the prostate is diagnosed.

8. The method according to any one of Claims 1 to 3, **characterised in that** the ratio is the following or the inverse ratio:

$$\frac{[ACT] \times [PSA - ACT]^2}{[free\ PSA]}.$$

9. The method according to Claim 8, **characterised in that**, when the ratio is higher than 17, and preferably higher than 40$(\mu g/ml)^2$, an adenocarcinoma of the prostate is diagnosed, and when the ratio is lower than 12, and preferably lower than 7$(\mu g/ml)^2$, a benign hypertrophy of the prostate is diagnosed.

10. A process using a method according to any one of Claims 1 to 9, **characterised in that** it consists in:

- determining the value of the parameters constituting the ratio which must be used,
- determining the value of the ratio,
- comparing the ascertained ratio with the reference values, and
- diagnosing either an adenocarcinoma of the prostate or a benign hypertrophy of the prostate, or finally an area of uncertainty implying the need for more extensive investigation, such as a biopsy, a digital rectal palpation or transrectal ultrasonography.

11. An immunological test enabling a process according to Claim 10 to be implemented, which uses:

• a free anti-PSA antibody and at least one other antibody selected from the anti-PSA total and anti-PSA-ACT antibodies, and
• an anti-ACT antibody.

12. A diagnostic kit enabling the diagnosis, prognosis or therapeutic monitoring of an adenocarcinoma of the prostate in a subject who is suspected to be suffering from said adenocarcinoma, or enabling differentiation between a benign pathology of the prostate and an adenocarcinoma of the prostate in a subject suspected to be suffering from one of these illnesses, said kit including:

• means for assaying the free PSA as a tissue marker and for assaying at least one other tissue marker selected from total PSA and PSA-ACT, and
• means for assaying the ACT as an inflammation marker.

13. The kit according to Claim 12, in which said means are antibodies.

**Patentansprüche**

1. Ein Analyseverfahren zur Diagnose, Prognose oder therapeutischen Betreuung eines Prostataadenokarzinoms oder einer gutartigen Prostatahypertrophie bei einem männlichen menschlichen Patienten, ohne eine Prostatabiopsie durchzuführen, **dadurch gekennzeichnet, dass** es für die Diagnose mindestens einen Gewebemarker, der aus freiem PSA, totalem PSA und PSA-ACT gewählt ist, und mindestens ACT als Entzündungsmarker verwendet.

2. Verfahren gemäß Anspruch 1, **dadurch gekennzeichnet, dass** es aus dem Verwenden der Konzentration von mindestens einem und vorzugsweise von mindestens zwei Gewebemarker(n), der/die in einem Verhältnis miteinbegriffen ist/sind, besteht, wobei die Gewebemarker aus Folgendem ausgewählt sind:

- [freiem PSA],
- [totalem PSA] und
- [PSA-ACT].

3. Verfahren gemäß Anspruch 1, **dadurch gekennzeichnet, dass** es aus der Verwendung der Konzentration des [ACT], das in einem Verhältnis als Entzündungsmarker miteinbegriffen ist, besteht.

4. Verfahren gemäß einem der Ansprüche 1 bis 3, **dadurch gekennzeichnet, dass** das Verhältnis das Folgende ist:

$$\frac{[ACT] \times [totales\ PSA]^2}{[freies\ PSA]}.$$

5. Verfahren gemäß Anspruch 4, **dadurch gekennzeichnet, dass** bei einer Verhältniszahl von mehr als 30 und vorzugsweise mehr als 50 $(\mu g/ml)^2$ ein Prostataadenokarzinom diagnostiziert wird und bei einer Verhältniszahl von weniger als 16,5 und vorzugsweise weniger als 12 $(\mu g/ml)^2$ eine gutartige Prostatahypertrophie diagnostiziert wird.

6. Verfahren gemäß einem der Ansprüche 1 bis 3, **dadurch gekennzeichnet, dass** das Verhältnis das Folgende ist:

$$\frac{[ACT] \times [PSA - ACT] \times [totales\ PSA]}{[freies\ PSA]}.$$

7. Verfahren gemäß Anspruch 6, **dadurch gekennzeichnet, dass** bei einer Verhältniszahl von mehr als 23 und vorzugsweise mehr als 45 $(\mu g/ml)^2$ ein Prostataadenokarzinom diagnostiziert wird und bei einer Verhältniszahl von weniger als 13,5, und vorzugsweise weniger als 9 $(\mu g/ml)^2$, eine gutartige Prostatahypertrophie diagnostiziert wird.

8. Verfahren gemäß einem der Ansprüche 1 bis 3, **dadurch gekennzeichnet, dass** das Verhältnis das Folgende oder das umgekehrte Verhältnis ist:

$$\frac{[ACT] \times [PSA\text{-}ACT]^2}{[freies\ PSA]}.$$

9. Verfahren gemäß Anspruch 8, **dadurch gekennzeichnet, dass** bei einer Verhältniszahl von mehr als 17 und vorzugsweise mehr als 40 $(\mu g/ml)^2$ ein Prostataadenokarzinom diagnostiziert wird und bei einer Verhältniszahl von weniger als 12 und vorzugsweise weniger als 7 $(\mu g/ml)^2$ eine gutartige Prostatahypertrophie diagnostiziert wird.

10. Ein durch ein Verfahren gemäß einem der Ansprüche 1 bis 9 verwendeter Vorgang, **dadurch gekennzeichnet, dass** er aus Folgendem besteht:

- Bestimmen des Werts der Parameter, die das Verhältnis, das verwendet werden soll, ausmachen,
- Bestimmen des Werts des Verhältnisses,
- Vergleichen des eruierten Verhältnisses mit den Referenzwerten und
- Diagnostizieren entweder eines Prostataadenokarzinoms oder einer gutartigen Prostatahypertrophie oder schließlich eines unklaren Bereichs, der ausführlichere Untersuchungen voraussetzt, wie etwa eine Biopsie, Fingerpalpation des Rektums, transrektale Sonographie.

11. Ein immunologischer Test zum Ermöglichen der Durchführung eines Vorgangs gemäß Anspruch 10, der Folgendes verwendet:

   • einen Antikörper des freien Anti-PSA und mindestens einen anderen Antikörper, der aus den Antikörpern des totalen Anti-PSA und des Anti-PSA-ACT gewählt ist, und
   • einen Anti-ACT-Antikörper.

12. Ein Diagnose-Kit, das die Diagnose, Prognose oder therapeutische Betreuung eines Prostataadenokarzinoms bei einer Person, von der vermutet wird, dass sie von diesem Adenokarzinom betroffen ist, ermöglicht, oder das das Unterscheiden zwischen einer gutartigen Prostatapathologie und einem Prostataadenokarzinom bei einer Person, von der vermutet wird, dass sie von einer dieser Krankheiten betroffen ist, ermöglicht, wobei das Kit Folgendes beinhaltet:

   • Mittel zum Dosieren des freien PSA als Gewebemarker und zum Dosieren mindestens eines anderen Gewebemarkers, der aus dem totalen PSA und dem PSA-ACT ausgewählt ist, und
   • Mittel zum Dosieren des ACT als Entzündungsmarker.

13. Kit gemäß Anspruch 12, wobei diese Mittel Antikörper sind.

Fig. 1

PSA libre / PSA total
(%)

PSA total
(ng/ml)

BPH
PCa

ACT (µg/ml)

Fig. 2

BPH
PCa

PSA total (ng/ml)

0.1    1.0    10.0    100.0    1000.0    10000.0

EP 1 295 125 B1

Fig. 3

EP 1 295 125 B1

$$\frac{ACT \times (PSA\ total)^2}{(PSA\ libre)}$$

Fig. 4

BPH □
PCa ●

PSA total (ng/ml)

EP 1 295 125 B1